Europäisches Patentamt

European Patent Office

Office européen des brevets

(11) Publication number: **0 103 955**
**A2**

(12) **EUROPEAN PATENT APPLICATION**

(21) Application number: **83304248.4**

(22) Date of filing: **21.07.83**

(51) Int. Cl.³: **A 61 K 9/06**
**A 61 K 31/425**

(30) Priority: **21.07.82 US 400215**

(43) Date of publication of application:
**28.03.84 Bulletin 84/13**

(84) Designated Contracting States:
**AT BE CH DE FR GB IT LI LU NL SE**

(71) Applicant: **Kligman, Albert M.**
**637 Pine Street**
**Philadelphia Pennsylvania 19106(US)**

(72) Inventor: **Kligman, Albert M.**
**637 Pine Street**
**Philadelphia Pennsylvania 19106(US)**

(74) Representative: **Shaw, Laurence**
**George House George Road**
**Edgbaston Birmingham B15 1PG(GB)**

(54) Treatment of athlete's foot.

㊿ A composition and method for the treatment of athlete's foot, the composition comprising by weight at least 2 percent thiabendazole and preferably 5 percent thiabendazole by weight. The method involves applying to the foot an effective amount of the aforesaid composition.

Croydon Printing Company Ltd.

ALBERT M KLIGMAN          1          AGENTS REF: 1071

## TREATMENT OF ATHLETE'S FOOT

This invention relates to an improved composition and method for treatment of athlete's foot.

The term "athlete's foot" refers to itching, scaling, toe-web lesions to which male athletes are particularly prone. The clinical spectrum of interdigital athlete's foot ranges from mild scaling to a painful, exudative, erosive, inflammatory process with fissuring. The most familiar type is characterised by scaly, soggy, whitish hyperkeratotic toe webs associated with pruritus and foul odour. Textbooks of dermatology discuss interdigital athlete's foot in the chapter on superficial fungus infections; various dermatophytes are cited as causative. The latest thinking about the nature of the disease can be seen in an article in the June, 1977 edition of Post Graduate Medicine (Volume 61, No. 6) entitled Interdigitial Athlete's Foot by Leyden and Kligman as well as an article appearing in the October, 1978 edition of Arch Dermatol (Volume 114) entitled Interdigital Athlete's Foot by Leyden and Kligman.

It is an object of this invention to provide an improved composition and method for the treatment of athlete's foot and like diseases. It is another object to provide a method and composition which shows an immediate improvement so encouraging the patient to continue with the treatment. It is another object to provide a composition and method by which a fungal infection is controlled, so reducing the risk of a secondary bacterial infection.

According to one aspect the invention provides a pharmaceutical composition characterised in that at least 2% thiabendazole and an effective amount of glycol are present.

Although thiabendazole is a known anti-fungicidal agent and glycols are known as anti-bacterial agents, so far as I am aware they have not been combined, certainly for the treatment of athlete's foot. No one else has hitherto appreciated that when such combination is used an almost immediate improvement will be noted by the patient. Prior art treatments may take up to seven days before any improvement is noted.

The concentration of thiabendazole is preferably at least about 2% by weight most preferably from 5% to 10% by weight.

The glycol may be propylene glycol, polyethylene glycol or mixtures. Propylene glycol, so far as is presently known is irritating and hence the concentration of propylene glycol must be kept at relatively known levels. Higher glycols, while less anti-bacterial are non-irritating. Also, the higher glycols add viscosity which is useful in making an effective formulation which will tend to stay in place on the foot.

Accordingly, it is preferred to use a glycol in a concentration which will provide anti-microbial actvity, be below the irritating level while achieving a suitable lotion quality (not a solution) for easy topical application and retention of the active ingredients.

The components may be mixed together, and possibly in a carrier, at room temperature. The glycol tends to act as a vehicle for the thiabendazole.

A preferred composition comprises (parts by weight):

        thiabendazole 5.0,
        polyethylene glycol 400 47.5,
        polyethylene glycol 4000 37.5,
        propylene glycol 10.0,
        arlacel 165 5.0 (a mixture of gylceryl
        monostearate and polyoxyethylene glycol stearate
        used as an emulsifier to create state emulsions
        in an acid system).

The invention is illustrated by the following evaluations:


Example I: effect of carrier


5.0% thiabendazole was evaluated in symptomatic interdigital athlete's foot (dermatophytosis complex) in various bases. The test agent was applied to one side, b.i.d., for three weeks; the opposite side was treated with a preparation containing 1% miconazole known as Micatin, a widely used anti-fungal cream. Groups of 7 and 10 subjects were used for each study.

    (a)  5% thiabendazole in Hydrophilic Ointment, vs. Micatin

Both sides cleared at about the same rate. Six of 9 subjects thought that the Micatin worked faster, especially in regard to relief of itching. Mycologic clearing (KOH) occurred more often with Micatin. Thus Micatin had a superior therapeutic effect.


In a preliminary study comparing 5.0% thiabendazole in Hydrophilic Ointment to 2.5 or so thiabendazole in the skin base the latter was clearly inferior; a 2.5% or so concentration was only moderately effective.

    (b)  5% Thiabendazole in Purpose Cream (Johnson & Johnson) vs Micatin

Both sides cleared at the same rate with excellent results. The subjects had no preference. Mycologic clearing was more frequent with Micatin.


### (c)    5% Thiabendazole in Lacticare Base (Stiefel Labs), vs. Micatin

5% thiabendazole was distinctly inferior to Micatlon in this base.  The results varied form poor to fair.  The Lacticare Base nuffifies the activity of thiabendazole.


### (d)    5% thiabendazole polyethylene glycol formula (hereinafter referred to as PEG) vs. Micatin

In this modified Carbowax vehicle, thiabendazole was appreciably better than Micatin.  Both sides cleared equally well after three weeks but symptomatic relief was swifter with PEG 200.  The smell diminished in a few days while with Micatin the smell took about a week to disappear.  The interspaces also tended to dry more speedily with thiabendazole.  The interspaces generally became mycologically negative (KOH mount) by three weeks.  Thiabendazole in this base was preferred by 8 of the 10 subjects in the study.  There were no adverse effects.

In a pilot study with 6 subjects, 2.5% thiabendazole in PEG 200 was compared to Micatin.  The clinical effects were similar, again with a speedier response to thiabendazole.  However, KOH examination at 3 weeks showed hyphal elements in 5 of 6 subjects on thiabendazole, compared to 2 of 6 on Micatin.

This and other trials have established that about 2.5% thiabendazole is below optimum. On the other hand, 10.0% concentrations seem to add very little to efficacy. It would appear that 5.0% thiabendazole is an appropriate concentration for maximal effects.

Example II

Studies with the Polyethylene Glycol

(a)  In vitro studies

These studies cover a two year period. It became evident as the work progressed that the vehicle itself was far from inert. These studies readily showed that PEG 200 (40%-50% aqueous) was bacteriostatic and bactericidal against the organisms which dominate the bacterial flora in interdigital athlete's foot, namely diptheroids and coagulase-negative cocci. Moreover, 50% concentrations were cidal for T. mentagraphytes and T. rubrum, the two fungi most often associated with athlete's foot. Likewise, Candida albicans and P. orbiculare were inhibited.

The anitmicrobial, anti-fungal and anti-yeast activity of PEG 200 was compared to propylene glycol. The effects were comparable: at concentrations higher than 25% both agents are strongly cidal.

(b)  In vivo studies with glycol alone

PEG 200 was compared to prolylene glycol in dermatophytosis complex.  The agents were applied to opposite sides twice daily for three weeks in eight subjects.

Both agents were quite effective in relieving smell, itching and "wetness".  The interspaces were greatly improved after three weeks though fungal elements were still present in most cases, albeit sparsely.  In two instances there was evidence of irritation with proplylene glycol.  One case with fissures was unable to continue treatment on the proplyene glycol side.  The latter is a known irritant in high concentrations.

PEG 200 itself was compared with proplyene glycol as regards irritancy in a 21-day, continuous occlusion test in 10 subjects.  Both agents were undiluted. Applications were made daily to the same sites.  The cumulative irritation score was considerably greater with propylene glycol.  PEG 200 is only a very mild irritant under these sever circumstances of continuous occlusion.

It is quite clear that the PEG 200 vehicle is by itself a rather effective modality for treating athlete's foot. It does not completely eratidcate fungi but does moderate the signs and symptoms of the disease, owing to its antibacterial powers.  This same vehicle was found to be useful in reducing the foul snmell of the feet of certain males with excessive sweating.  Bromsidrosis

0103955

(stinky feet) is mainly due to overgrowth of resident diphtheroids in hydrated horny layers.

The PEG 200 vehicle completely suppressed the microbial flora induced by covering the forearm with Saran Wrap for 48 hours. In six hours the density of resident orgamisms fell from millions to less than hundreds per square centimeter.

### (c)  5% Thiabendazole in PEG versus Lotromin*

The test agents were applied twice daily to opposite feet of 21 males (two separate studies) for three weeks. All had severe dermatophytosis complex. *Lotromin is a preparation containing clotrimazole.

The results were comparable. Both materials were highly effective, with mycologic and clinical clearing in nearly all cases. Symptomatic relief was again faster with thiabendazole, but the difference was not nearly as noticeable as with Micatin. Lotromin is regarded as superior to Micatin in athlete's foot. Lotromin also inhibits bacteria but, being insoluble, acts more slowly. PEG is also modestly keratolytic and enhances penetration of thiabendazole.

The PEG has two characteristics which enhance its value as a vehicle. It is anti-bacterical and it reduces the thickness of the horny layer, (keratolytic) hence promoting penetration of the active medicament.

0103955

In eight cases, followed for three weeks after the last application, relapse seemed to occur more quickly with Lotromin. Thiabendazole in PEG base is regarded as probably superior to Lotromin.

(d)   5% Thiabendazole in PEG 200 Base versus Micatin against Tinea Versicolor

A comparison was made on opposite sides of the back of 7 males with extensive tinea versicolor. Applications were made twice daily for two weeks.

With both agents, the lesions cleared (save for pigmentation) by two weeks. Scrapings examined in KOH mounts failed to show organisms.

Tinea versicolor responds rapidly to 5.0% thiabendazole in PEG 200.

(e)   5% Thiabendazole in PEG 200 versus Micatin against Pitted Keratolysis

Eleven athletic males with extensive pitted keratolyis were treated on opposite sides twice daily of one month. All had intense bromidrosis.

The pits resolved on both sides at about the same rate in all subjects, taking almost four weeks to disappear. The smell abated within a few days of treatment with thiabendazole. With Micatin the smell lasted for at least a week.

Both agents are very effective in this disorder but 5% thiabendazole was faster.

Example III

Miscellaneous

(a)  Seven cases of tinea cruris have been treated with 5.0% thiabendazole in PEG 200 base. Applications were twice daily for three weeks.

In every instance, the lesions had almost regressed by two weeks; the active borders were no longer elevated and red.  The lesions were mycologically negative (KOH examination) by three weeks.  This response is comparable to Lotromin.

(b)  thirteen cases of erythrasma (9 of feet, 4 of groin and axillae) received 5.0% thiabendazole twice daily for two weeks.

In all cases fluorescence became negative by two weeks. Gram-stained scrapings failed to show C. minutissimum. This formulation causes rapid regression of erythrasma.

(c)  Three cases of extensive T. rubrum infection of the trunk have been treated with successful clearing after 4 weeks of twice daily applications.

(d)   5.0% thiabendazole in PEG vehicle has been ineffective in three cases of onychomycosis of the finger nails treated by overnight occlusion.

Other tests were run with reference to ringworm of the foot.

## Procedure

Sixteen college student male athletes with long standing interdigital, bilateral ringworm of the feet completed a 3 week study.

These subjects had rather severe, ringworm of the complex type with maceration, foul smell, scaling and pruritus.  All were KOH positive.  Fungus was recovered in culture.  (Seven were T. mentagrophytes).

The test agents were applied twice daily for 3 weeks to opposite feet.  These were 2% Miconazole Cream (J & J Micatin) and 5% Thiabendazole in PEG vehicle.

Clinical rating was done weekly with a one week follow-up after the last treatment.

## Results and Conclusions:

Both agents were quite effective.  Thiabendazole was clinically and mycologically superior to Miconazole.
Symptomatic relief occurred more swiftly; the signs of
the disease, especially maceration also regressed
faster.

No adverse side effects were noted with either cream.

Thiabendazole brought about swifter resolution of the
signs and symptons of athletes foot in comparison to
Miconazole.  This formulation ranks foremost of all the
anti-fungals we have tested.  It is far superior to
tolnaftate and haloprogen.  It is also better than
chlortrimazole (Lotromin).

In dermatophytosis simplex (not reported here)
Thiabendazole was equivalent to but not better than
miconazole or chlortrimazole.  The simplex type is not
complicated by bacterial overgrowth, is mostly
asymptomatic and is more responsive to topical
treatment.

ALBERT M KLIGMAN          13          AGENTS REF: 1071

## 5% Thiabendazole vs Miconazole

## Clinical Assessments on Interdigital Ringworm

| Subject | Thiabendazole | | | Micatin | | |
|---|---|---|---|---|---|---|
| | Week 1 | Week 2 | Week 3 | Week 1 | Week 2 | Week 3 |
| 1 | P | G | G | P | F | G |
| 2 | F | G | E | P | F | E |
| 3 | F | G | E | F | G | E |
| 4 | P | G | E | P | F | G |
| 5 | F | F | G | F | G | G |
| 6 | F | G | G | P | F | E |
| 7 | G | G | E | F | G | G |
| 8 | F | G | E | P | G | G |
| 9 | G | G | G | F | G | E |
| 10 | F | G | E | P | F | E |
| 11 | F | G | E | P | G | E |
| 12 | F | G | E | P | F | E |
| 13 | F | F | G | P | G | G |
| 14 | G | E | E | P | G | G |
| 15 | F | G | G | F | E | G |
| 16 | P | F | F | P | F | F |

Key

   P = Poor

   F = Fair, less itchy, less macerated but still
wet odourous and active.

        G = Good, odour suppressed, no pruritus,
dry, slight whiteness.

   E = Excellent, clinically resolved except for
slight scaling.

ALBERT M KLIGMAN                14          AGENTS REF: 1071

## 5% Thiabendazole vs Miconazole

## Mycologic Assessment

## (All KOH positive before Treatment)

| Subject | Thiamendazole | | | Micatin | | |
|---|---|---|---|---|---|---|
| | Week 1 | Week 2 | Week 3 | Week 1 | Week 2 | Week 3 |
| 1 | ++ | + | 0 | ++ | + | 0 |
| 2 | ++ | + | 0 | ++ | + | + |
| 3 | + | 0 | 0 | ++ | + | 0 |
| 4 | +++ | + | + | +++ | ++ | + |
| 5 | + | 0 | 0 | + | + | 0 |
| 6 | + | 0 | 0 | + | 0 | 0 |
| 7 | + | + | 0 | ++ | + | + |
| 8 | ++ | + | + | ++ | + | + |
| 9 | + | + | 0 | ++ | + | 0 |
| 10 | ++ | + | + | ++ | + | 0 |
| 11 | + | 0 | 0 | + | 0 | + |
| 12 | + | 0 | 0 | + | 0 | 0 |
| 13 | + | + | 0 | ++ | + | 0 |
| 14 | + | 0 | 0 | ++ | + | + |
| 15 | + | + | 0 | + | + | 0 |
| 16 | +++ | ++ | + | ++ | + | + |

Key

+ = filaments sparse

++ = filaments in moderate abundance

+++ = numerous filaments

Example IV

Eight subjects with symptomatic, bilateral foot ring
worm were treated on one side with the formulation
involving 5% thiobendazole in a glycol base.
Application of the foregoing to the one side occurred
twice daily for three weeks.  On the other side a glycol
by itself was used similarly.

Both sides initially improved considerably.  The smell
and maceration improved appreciably within a few days,
there being little difference by the end of the first
week.  Thereafter, the combination thiobendazole-glycol
showed greater efficacy in reducing scaling and
maceration.

Of greater significance, however, was the result of
examining KOH mounts of scales for fungi at the end of
three weeks.  Seven of the eight subjects were KOH
negatives in the thiobendazole side.  Only two of the
eight showed absence of fungi with the glycol alone.

Thus it is clear that the glycol is not effective in
eliminating fungi even though it moderates the
symtomatology  because of its anti-bacterial activity.
I have discovered that an effective treatment must also
contain an anti-fungial agent which is active in vivo.
The glycol vehicle by itself is incapable of eliminating
the fungi located deeply in the horny layer.  Hence, a quick recurrence is inevitable with the vehicle alone.

Three weeks later four of the subjects were seen.  On the glycol side there was almost complete relapse. However, the thiobendazole-glycol side was clear.

5% thiobendazole in polyethylene glycol 400 was used as compared with polyethylene glycol 400 by itself in five subjects.

The results obtained were comparable to those above. None of the five subjects became KOH negative with the polyethylene glycol 400 alone.  Four of the five were negative on the thiobendazole-polyethylene glycol 400 side.

Again symptomatic improvement occured on both sides during the first week.  Thereafter, the thiobendazole-polyethylene glycol 400 side was unequivocally more effective in producing clearing.

From all of the foregoing it will be seen that the present invention recognizes that it is the fungus which is the primary invader, causing changes which permit the bacteria to penetrate and overgrow.  It should be kept in mind that so long as the fungus is controlled, and assuming there are no breaks in the skin, the bacteria normally found in the foot will not invade the foot to cause athlete's foot.  It is only when the fungus achieve penetration of the protective skin that the normal bacteria can penetrate to cause the undesirable effects of athlete's foot.  It will be seen that the present invention strikes at both aspects of athlete's foot, the primary fungus and the secondary bacteria.

CLAIMS

1. A composition for the treatment of athlete's foot characterised by at least 2% by weight of thiabendazole and effective amount of a glycol.

2. A composition according to Claim 1, characterised in that the glycol is polyethylene glycol.

3. A composition according to Claim 1 or 2 characterised in that the glycol is propylene glycol and polyethylene glycol.

4. A composition according to any preceding Claim characterised in that thiabendazole is present in range of 2% to 10% by weight.

5. A composition according to any preceding Claim characterised in that the composition comprises by weight:

        thiabendazole (5.0%)
        polyethylene glycol 400 (47.5%)
        polyethylene glycol 4000 (37.5%)
        propylene glycol (10%)
        arlacel 165 (5.0%)

6. A method for treatment for athlete's foot characterised by applying to the foot an effective amount of a composition having by weight at least 2% thiabendazole and an effective amount of a glycol.

7.  A method according to Claim 6 characterised in that the thiabendazole is present in an amount of 5.0% by weight.

8.  A method according to Claim 6 characterised in that the glycol is polyethylene glycol.

9.  A method according to Claim 7 characterised in that the glycol comprises propylene glycol and polyethylene glycol.

10.  A method according to Claim 6 characterised in that the composition comprises (by weight)

        thiabendazole (5.0%)
        polyethylene glycol 400 (47.5%)
        polyethylene glycol 4000 (37.5%)
        propylene glycol (10.0%)
        arlacel 165 (5.0%)